# EUROPÄISCHE PATENTSCHRIFT

(11) **EP 2 949 325 B1**
(45) Veröffentlichungstag und Bekanntmachung des Hinweises auf die Patenterteilung: **03.08.2022**
(21) Anmeldenummer: 14170308.2
(22) Anmeldetag: 28.05.2014
(51) Int. Cl.: A61K 31/375, A61P 31/12, A61P 31/10, A61P 31/04, A61K 33/38, A61K 33/24, A61K 9/00, A61L 15/18, A61L 15/46, A61L 26/00, A61P 31/22, A61P 43/00

(54) **Verwendung einer antimikrobiellen Zusammensetzung**
Use of an antimicrobial composition
Utilisation d'une composition antimicrobielle

(43) Veröffentlichungstag der Anmeldung: 02.12.2015
(73) Patentinhaber: AgXX Intellectual Property Holding GmbH, 14169 Berlin (DE)
(72) Erfinder: Landau, Uwe, 14169 Berlin (DE); Lauenroth, Michael, 14129 Berlin (DE)
(74) Vertreter: Remus, Alvaro Johannes

(56) Entgegenhaltungen:
- WO-A2-2008/046513
- DE-A1-102012 218 233
- Nsánchez-Najera Rosa Isela 1 ET AL: "Ascorbic Acid On Oral Microbial Growth and Biofilm Formation", The Pharma Innovation, 1. Juni 2013 (2013-06-01), XP055125663, New Delhi Gefunden im Internet: URL:http://search.proquest.com/docview/136 6365936 [gefunden am 2014-07-02]

## Beschreibung

### Hintergrund der Erfindung

Die Erfindung betrifft die Verwendung einer antimikrobiellen Zusammensetzung zur topischen Behandlung oder Vermeidung von Erkrankungen der Haut, Hautanhangsgebilde oder Schleimhaut, die durch die Infektion mit mindestens einem Herpes simplex-Virus verursacht werden. Die Erfindung betrifft ferner ein Verbandmaterial oder einen Wundschnellverband, das oder der eine antimikrobielle Zusammensetzung umfasst.

### Stand der Technik

Aus der WO 2008 046513 A2 ist eine bioaktive, Silber, Ruthenium und ein Vitamin enthaltende metallische Beschichtung bekannt, die zur Entkeimung, Desinfektion und Dekontamination von Wasser oder wässrigen Lösungen verwendet wird. Die Kombination von Silber mit Ruthenium und einem Vitamin oder dessen Derivat führt zu einer schnelleren und effizienteren Abtötung von Mikroorganismen. Gleichzeitig verhindern diese bioaktiven Metalloberflächen die Besiedelung mit Mikroorganismen und die Anheftung oder stabile Ablagerung von problematischen Biomolekülen wie zum Beispiel DNA, RNA oder Proteinen. Durch die Beschichtung entsteht eine sich selbst reinigende Oberfläche, die bei Kontakt mit Wasser oder wässrigen Lösungen sehr schnell und effizient dessen Keimfreiheit herstellt und über längere Zeiträume aufrechterhält. Antibiotika-Resistenzen vieler pathogener Bakterien stellen weltweit ein schwerwiegendes Problem bei der Behandlung von Patienten dar, für das es derzeit häufig keine zufriedenstellenden Lösungen mehr gibt. Insbesondere urologischen Infektionen und Infektionen der Haut verursachen dabei die größten Probleme (WHO Library Cataloguing-in-Publication Data (2014): Antimicrobial resistance: global report on surveillance, World Health Organization, ISBN 978 92 4 156474 8). Es besteht daher ein immer größer werdender Bedarf an Therapien zur Behandlung von bakteriellen Infektionen, die ohne Antibiotika auskommen.

Aber auch bei viralen Infektionen fehlen häufig Alternativen zu den bereits bekannten Therapien. Ein Beispiel hierfür sind Herpes-Infektionen. Mehr als 85% aller Deutschen sind mit dem Herpes simplex-Virus Typ 1 (HSV-1) infiziert, bei etwa 12 Millionen Betroffenen bricht die Erkrankung regelmäßig aus (ca. 20 %). Für andere Länder dürften die Zahlen aus Deutschland in etwa übertragbar sein. Herpesviren gelten weltweit als die am weitesten verbreiteten Viren. Der Lippenherpes zählt in Deutschland zu den häufigsten Erkrankungen der Haut. Die Erstinfektion verläuft meist unbemerkt als Tröpfchen- oder Schmierinfektion vor dem 6. Lebensjahr. Nach der Erstinfektion bleibt das Virus lebenslang im Körper des Betroffenen. Der Ausbruch von Herpes dauert ca. 10 - 14 Tage und verläuft in 5 verschiedenen Stadien: Juckreizstadium, Bläschenstadium, Nässe/Wundstadium, Krustenstadium und Heilungsstadium.

Derzeit wird weltweit bei der Behandlung der Herpes simplex-1-Erkrankung auf zwei unterschiedliche Strategien gesetzt: Virostatika (Salben, Tabletten, Suspensionen) und Herpespflaster mit Inhaltsstoffen zur Reduzierung der Beschwerden beim Krankheitsverlauf. Bekannte Wirkstoffe sind beispielsweise Aciclovir und Penciclovir. Beide Wirkstoffe sind in Form von Cremes und Tabletten erhältlich und hemmen den Stoffwechsel der Zelle, wobei der Wirkstoff nur in infizierten Zellen aktiviert wird. Herpespflaster arbeiten auf der Grundlage der Hydrokolloid-Therapie und werden auf die entsprechende Erkrankungsstelle appliziert. Sie müssen gut haften und sollen möglichst wenig sichtbar sein. Gleichzeitig sind auf dem Pflaster Stoffe aufgebracht, die den Krankheitsverlauf erleichtern und Symptome wie Juckreize, Schwellungen und Rötungen reduzieren.

Die bisher eingesetzten Behandlungsmethoden und Wirkstoffe können jedoch den Krankheitsverlauf nicht entscheidend beeinflussen, sondern nur die unangenehmen Symptome des Lippenherpes lindern. Es wird daher immer noch ein Therapieansatz gesucht, der den Ausbruch der Herpeserkrankung gleich nach deren ersten Auftreten (Kribbeln, Hautspannung oder Schwellung) zurückdrängt und die Bläschenbildung als nachfolgendes Stadium gar nicht erst eintreten lässt.

### Beschreibung der Erfindung

Der Erfindung liegt die Aufgabe zugrunde, alternative Wirkstoffe und/oder Materialien zur topischen Behandlung oder Vermeidung von Erkrankungen der Haut, Hautanhangsgebilde oder Schleimhaut, die durch die Infektion mit mindestens einem Herpes simplex-Virus verursacht werden, bereitzustellen.

Diese Aufgabe wird erfindungsgemäß durch die entsprechende therapeutische und präventive Verwendung einer antimikrobiellen Zusammensetzung gelöst, welche metallisches Silber und metallisches Ruthenium sowie mindestens ein Vitamin oder mindestens ein Derivat eines Vitamins umfasst. In der vorliegenden Erfindung ist vorgesehen, dass das Vitamin Ascorbinsäure oder ein Salz der Ascorbinsäure ist. Es hat sich überraschender Weise herausgestellt, dass eine Kombination der Edelmetalle Silber und Ruthenium, die mittels eines Vitamins aktiviert wurde, eine keimabtötende Wirkung auf alle getesteten Mikroorganismen hat und sich hervorragend zur topischen Behandlung von entsprechenden Infektionen der Haut, Hautanhangsgebilde oder Schleimhäute eignet. Dies gilt sowohl für den Menschen als auch für Tiere. Dabei können durch die topische Anwendung der antimikrobiellen Zusammensetzung einerseits durch Mikroorganismen verursachte Erkrankungen schnell und schonend kuriert und andererseits gefährdete Hautbereiche, wie z. B. offene Wunden, zur Vermeidung von Infektionen sehr effektiv präventiv behandelt werden. Die metallisches Silber und metallisches Ruthenium sowie mindestens ein Vitamin oder mindestens ein Derivat eines Vitamins umfassende Zusammensetzung wirkt dabei spezifisch gegen die Mikroorganismen und ist für die behandelten Haut- oder Schleimhautzellen sowie Hautanhangsgebilde unbedenklich.

Der Mikroorganismus kann beispielsweise ein Virus, ein Bakterium oder ein Pilz sein. Die Wirksamkeit der antibakteriellen Zusammensetzung gegen diese Arten von Mikroorganismen und ggf. deren Sporen-Stadien konnte in zahlreichen Tests nachgewiesen werden. Wie in einer Untersuchung an der antibakteriellen Zusammensetzung und Silber von 13 Mikrobiologen aus Deutschland und Spanien mit 29 verschiedenen Bakterien gezeigt worden ist, wirkt die antibakterielle Zusammensetzung, anders als z. B. Silber, sehr effizient auch gegen Antibiotika resistente Bakterien und verhindert äußerst effizient die Bildung von Biofilmen. Wie unter näher ausgeführt, wirkt die antibakterielle Zusammensetzung auch gegen Pilze und sogar Algen.

Der Mikroorganismus kann beispielsweise auch das Herpes simplex-Virus Typ 1 (Humanes Herpesvirus 1 = HSV-1) sein, das in erster Linie als Erreger des Lippenherpes (Herpes labialis) bekannt ist. Aber auch etwa 20-30% der genitalen Herpes-Infektionen (Genitalherpes = Herpes genitalis) werden durch HSV-1 verursacht. Ohne die Behandlung eines Lippenherpesausbruchs beträgt die Zeit bis zur Abheilung 10 -14 Tage. Studien haben gezeigt, dass herkömmliche Herpes-Cremes den Heilungsverlauf nicht oder nur geringfügig verkürzen (0,5-2,0 Tage). Herpesbläschen an den Lippen bzw. im Gesicht stellen aber für den Erkrankten eine physische, psychische und soziale Belastung dar. Daher kommt der Verkürzung des Krankheitsverlaufs für den Patienten die höchste Priorität zu. Die metallisches Silber und metallisches Ruthenium sowie mindestens ein Vitamin oder mindestens ein Derivat eines Vitamins umfassende antibakterielle Zusammensetzung wirkt nachweislich derart effizient gegen das Herpes simplex-Virus Typ 1, dass es den Herpes innerhalb eines Tages nach dem Auftreten der ersten Symptome vollkommen zurückdrängt. Im Gegensatz zu den bereits am Markt verfügbaren Herpes-Behandlungs-Lösungen, kann die Herpeserkrankung durch die antimikrobielle Zusammensetzung um mehrere Tage (8,5 - 12 Tage) verkürzt werden.

Der Mikroorganismus kann aber beispielsweise auch das Herpes simplex-Virus Typ 2 (Humanes Herpesvirus 2 = HSV-2) sein, das für die überwiegenden Mehrzahl der genitalen Herpes-Manifestationen (am Penis, an der Scheide und im Analbereich) verantwortlich ist. Die metallisches Silber und metallisches Ruthenium sowie mindestens ein Vitamin oder mindestens ein Derivat eines Vitamins umfassende antibakterielle Zusammensetzung wirkt ebenfalls sehr effizient gegen das Herpes simplex-Virus Typ 2 und ist daher auch ein effektives Mittel gegen Genitalherpes (Herpes genitalis) .

In vorteilhafter Ausgestaltung der Erfindung ist vorgesehen, dass die antimikrobielle Zusammensetzung auf mindestens eine Oberfläche eines Trägermaterials aufgebracht ist. Das Trägermaterial kann beispielsweise ein Gewebe, eine Folie, ein Netz, ein Verbandmaterial, eine Kompresse oder ein Wundschnellverband ("Pflaster") sein. Das Trägermaterial kann ferner beispielsweise zumindest teilweise aus Metall, Kunststoff, Glas, Keramik, Naturfasern, Kunstfasern oder anderen geeigneten Materialen bestehen.

Das Trägermaterial kann beispielsweise so beschaffen sein, dass es benetzbar und/oder durchlässig für Feuchtigkeit ist. Feuchtigkeit fördert bzw. steigert die Wirkung der antimikrobiellen Zusammensetzung, so dass es von Vorteil ist, wenn das Trägermaterial Feuchtigkeit aufnehmen und praktisch als Feuchtigkeitsspeicher dienen kann. Zusätzlich oder alternativ kann das Trägermaterial auch so beschaffen sein, dass Feuchtigkeit durchlässt, so dass die antimikrobielle Zusammensetzung und/oder die zu behandelnde Haut/Schleimhautoberfläche von außen mit Feuchtigkeit benetzt werden kann, obwohl sie von dem Trägermaterial bedeckt ist/sind.

Das Trägermaterial kann in vorteilhafter Ausgestaltung der Erfindung auch ein Material zur Speicherung von Feuchtigkeit umfassen, beispielsweise Wasser enthaltende, aber wasserunlösliche Polymere (sogenannte "Hydrogele").

In weiterer vorteilhafter Ausgestaltung der Erfindung ist vorgesehen, dass das Trägermaterial gas- und/oder luftdurchlässig ist, da beispielsweise Sauerstoff für die Wirksamkeit der antibakteriellen Zusammensetzung von besonderem Vorteil ist.

In weiterer vorteilhafter Ausgestaltung der Erfindung ist vorgesehen, dass die antimikrobielle Zusammensetzung mindestens eine Silberschicht mit mindestens einer darauf aufgebrachten Ruthenium-Schicht umfasst, wobei die Silberschicht von der Ruthenium-Schicht in mindestens einem Teilbereich unbedeckt ist. So kann die Ruthenium-Schicht beispielsweise in Form von Inseln oder Nestern, sozusagen Cluster-förmig, auf der Silberschicht aufgebracht sein. Hierdurch kann eine optimale Wirksamkeit der antibakteriellen Zusammensetzung gewährleistet werden.

Die antibakterielle Zusammensetzung kann beispielsweise durch das Aufbringen einer Ruthenium-Schicht auf eine silberhaltige Oberfläche oder ein silberhaltiges Trägermaterial, oder das Aufbringen einer Silber-Beschichtung auf das Trägermaterial und anschließendes Aufbringen einer Ruthenium-Schicht auf die Silber-Beschichtung, oder das Aufbringen von Ruthenium-Silber-Partikeln auf eine Oberfläche oder das Trägermaterial, hergestellt werden. Die derart erzeugte Ag/Ru-Oberfläche wird dann durch das Aufbringen von mindestens einem Vitamin oder mindestens einem Derivat eines Vitamins aktiviert. Die Schichten, d.h. die Ruthenium-Schicht und eine ggf. erforderliche Silberunterschicht (i.d.R. mit einer Dicke von 2-10 µm), werden vorzugsweise galvanisch aufgebracht oder abgeschieden. Andere Beschichtungsverfahren, wie PVD-, CVD-, Sputter-, Sol-Gel- und Reduktionsverfahren, sind ebenfalls hierfür geeignete Plattierverfahren.

In weiterer vorteilhafter Ausgestaltung der Erfindung ist vorgesehen, dass die antimikrobielle Zusammensetzung mindestens einen Silberdraht oder zumindest silberbeschichteten Draht umfasst, der teilweise mit einer Ruthenium-Schicht bedeckt ist. Anstelle eines Silberdrahts kann beispielsweise auch ein silberbeschichteter Edelstahldraht verwendet werden, der dann teilweise mit Ruthenium beschichtet wird. Anschließend wird dieser Ag/Ru-Draht dann durch das Aufbringen von mindestens einem Vitamin oder mindestens einem Derivat eines Vitamins aktiviert. Die drahtförmige antimikrobielle Zusammensetzung kann dann beispielsweise als Bestandteil eines Netzes oder Siebes verarbeitet und verwendet werden.

Bei der mit Ruthenium zu beschichtenden Oberfläche, die beispielsweise eine silberhaltige Oberfläche oder Silberoberfläche umfassen kann, kann es sich um ein beliebiges Material (wie z.B. Metall oder Kunststoff) mit einer aufgebrachten, dünnen, silberhaltigen Beschichtung handeln, die zusammen mit der aufgebrachten äußeren Ruthenium-Schicht ein wirksames Ruthenium-Silber-Sandwich-System mit einer Silber- oder Silberlegierungs-Unterschicht oder - Unterlage und einer feuchtigkeits- oder nässedurchlässigen Ruthenium-Außenschicht, -Oberschicht oder -Deckschicht bildet.

In weiterer vorteilhafter Ausgestaltung der Erfindung ist vorgesehen, dass die antimikrobielle Zusammensetzung Silber-Ruthenium-Bimetall-Partikel umfasst, die mit dem Vitamin bzw. Vitaminderivat nachbehandelt wurden. Solche Partikel können beispielsweise als Bestandteil eine Pulvers, einer Suspension, einer Creme, einer Salbe oder eines Gels eingesetzt werden.

In weiterer vorteilhafter Ausgestaltung der Erfindung ist vorgesehen, dass die antimikrobielle Zusammensetzung, welcher Form auch immer, in einer Suspension, einer Creme, einer Salbe, einem Gel oder einem Pulver enthalten ist und zur topischen Behandlung von mikrobiellen Erkrankungen der Haut, Hautanhangsgebilde oder Schleimhaut eines Menschen oder eines Tieres verwendet wird.

In weiterer vorteilhafter Ausgestaltung der Erfindung ist vorgesehen, dass das Vitamin mindestens eine Verbindung aus der Gruppe der wasserlöslichen Vitamine oder ein Salz oder saures Derivat dieser Verbindung ist. Bei dieser Ausgestaltung der Erfindung wird, anders als beim bekannten oligodynamischen Effekt des Silbers, die antimikrobielle Wirkung der Zusammensetzung aufgrund eines völlig anderen Wirkmechanismus erheblich verbessert bzw. durch die Kombination von Silber mit Ruthenium und einem Vitamin, beispielsweise Ascorbinsäure oder deren Derivaten, erheblich verstärkt. Die antibakterielle Zusammensetzung führt zu einer schnelleren und effizienteren Abtötung von Mikroorganismen. Gleichzeitig verhindern solche antibakteriellen Zusammensetzungen die Infektion empfindlicher oder geschwächter Haut- und Schleimhautoberflächen mit Mikroorganismen und die Anheftung oder stabile Ablagerung von Biomolekülen, wie DNA, RNA oder Proteinen auf der Haut oder Schleimhaut.

Beispielsweise kann eine Silber-/Ruthenium-Oberfläche mit mindestens einem wasserlöslichen Vitamin behandelt werden bzw. umfasst den oder die zusätzlichen Stoffe, so dass die Oberfläche durch diese Stoffe aktiviert wird und eine unerwartet starke antimikrobielle Wirkung der Zusammensetzung eintritt. Überraschender Weise hat sich herausgestellt, dass nur ein nicht abgebautes wasserlösliches Vitamin die unerwartete antimikrobielle Wirkung im Zusammenspiel mit Silber und Ruthenium bewirkt. Aus vielen Messungen hat sich ergeben, dass sich durch die Nachbehandlung einer mit Ruthenium beschichteten Silberoberfläche mit z. B. Ascorbinsäure die Oberfläche signifikant verändert und die antimikrobielle Wirkung nach einem ganz anderen Mechanismus als bei der klassischen Silbertechnologie (klassische, oligodynamische Silbertechnologie = Abgabe von Silberionen) abläuft. Es gibt Hinweise darauf, dass die Wirkung der antimikrobiellen Zusammensetzung von einem mikroelektrischen Feld ausgeht und das Benetzungsverhalten der Oberfläche beispielsweise deutlich verändert ist, wenn man eine reine Silber-/Ruthenium-Oberfläche mit der aktivierten antimikrobiellen Zusammensetzung vergleicht. Bei EM-Atom-Kraft-Mikroskop-Messungen wurden ebenfalls entscheidende Unterschiede festgestellt, die einen deutlichen Hinweise darauf geben, dass sich durch die Kombination von Silber/Ruthenium mit einem wasserlöslichen Vitamin ein mikroelektrisches Feld aufbaut, das bei reinen Silber-/Ruthenium -Oberflächen nicht beobachtet wird. Es kommt dabei zu einer kathodischen Sauerstoff-Reduktion, die zu einer Bildung freier Sauerstoffradikale ("Reaktive Sauerstoffspezies"; englisch: reactive oxygen species = ROS) wie beispielsweise H₂O₂ führt. Gleichzeitig bildet sich durch die antibakterielle Zusammensetzung eine chemisch sehr stabile Oberfläche aus, die z. B. gegen Sulfide und Chloride reaktionsfest ist. Die hohe Stabilität gepaart mit einer langanhaltenden, starken antimikrobiellen Wirksamkeit ist durch mehrjährige antimikrobielle Einsätze der antibakterielle Zusammensetzung in diversen wässrigen Lösungen (z.B. Kühlschmiermittellösungen, Prozesswässern mit Korrosionsschutzmitteln und Emulgatoren der unterschiedlichsten Art oder Urin) bestätigt worden.

Die Aufgabe wird erfindungsgemäß ferner durch Verbandmaterial oder einen Wundschnellverband gelöst, das oder der eine antimikrobielle Zusammensetzung umfasst, welche metallisches Silber und metallisches Ruthenium sowie Ascorbinsäure oder ein Salz der Ascorbinsäure umfasst. Ein solcher Verband bzw. ein solches Pflaster eignet sich in vorteilhafter Weise für das Applizieren und topische Anwenden der antimikrobiellen Zusammensetzung zur effektiven Behandlung von durch pathogene Mikroorganismen verursachten Erkrankungen der Haut, Hautanhangsgebilde oder Schleimhaut sowie zum Schutz gefährdeter Hautbereiche, wie z. B. offenen Wunden, vor entsprechenden Infektionen .

"Topische Behandlung" im Sinne der Erfindung bezeichnet die Anwendung von Wirkmechanismen dort, wo sie therapeutisch wirken sollen, im Gegensatz zur sogenannten systemischen Behandlung, d. h. der Gabe von Arzneimitteln beispielsweise als Infusion oder Tablette. Die topische Behandlung stellt also eine lokale Therapieform dar, die insbesondere in der Dermatologie, der Augenheilkunde, der Hals-Nasen-Ohrenheilkunde und der Frauenheilkunde angewendet wird, d. h. in Bereichen des Körpers, die von außen zugänglich sind, wie beispielsweise Haut, Hornhaut (des Auges) oder Schleimhäute. Topische Behandlungsformen haben den Vorteil, dass die Wirkstoffe oder Wirkmechanismen nur dort wirken, wo sie benötigt werden, während die gesunde Haut bzw. Schleimhaut geschont wird. Durch die lokale Anwendung können darüber hinaus viele systemische Nebenwirkungen vermieden werden.

Der Begriff "Haut" im Sinne der Erfindung umfasst das äußere Organ eines menschlichen oder tierischen Körpers, das u. a. der Abgrenzung des Körperinneren nach Außen dient. Der Begriff "Haut" umfasst dabei alle Schichten des Organs, insbesondere Epidermis (Oberhaut), Dermis (Lederhaut) und Subcutis (Unterhaut). Im Sinne der Erfindung umfasst der Begriff "Haut" insbesondere auch die Oberflächen der Lippen und Schamlippen sowie des Außen- und Mittelohrs.

Der Begriff "Hautanhangsgebilde" im Sinne der Erfindung umfasst die Haare mit ihren Talgdrüsen und dem Haarbalgmuskel (Musculus arrector pili), Nägel, Hörner und Schweißdrüsen (einschl. Milchdrüsen) eines menschlichen oder tierischen Körpers.

Der Begriff "Schleimhaut" im Sinne der Erfindung umfasst die Schutzschichten (lat.-med. Tunica mucosa), die das Innere von Hohlorganen eines menschlichen oder tierischen Körpers auskleiden und Schleimstoffe (Mucinen) produzieren bzw. absondern, insbesondere auch die Bindehaut des Auges, die Mundschleimhaut, die Deckschicht der Eichel und die Schleimhaut der Vagina.

Die Erfindung wird im Folgenden anhand der Abbildungen beispielhaft näher erläutert.

### Kurze Beschreibung der Abbildungen

Es zeigt:
**Figur 1** eine fotographische Abbildung einer mit der antimikrobiellen Zusammensetzung beschichteten selbstklebenden Folie mit Hydrogel-Untergrund (Feuchtigkeitsspeicherung);
**Figur 2** eine fotographische Abbildung eines applizierten Trägermaterials mit antimikrobieller Zusammensetzung;
**Figur 3** fotographische Abbildungen des Heilungsverlaufs eines Lippenherpes unter der Behandlung mit der antimikrobiellen Zusammensetzung;
**Figur 4** fotographische Abbildungen von Nährböden mit Mikroorganismus-Kolonien rund um mit der antimikrobiellen Zusammensetzung beschichteten Trägermaterialien ("AgXX" = aktivierte Ag/Ru-Zusammensetzung),
   a) Escherichia coli (zusätzlich Silber ("Ag") als Kontrolle),
   b) Candida parapsilosis,
   c) Penicillium notatum;
**Figur 5** mikroskopische Abbildungen von Hefezellen nach 15 Minuten Einwirkdauer einer pulverförmigen antibakteriellen Zusammensetzung und von Nano-Silber (dunkle Bereiche),
   a) aktivierte Ag/Ru-Zusammensetzung,
   b) Nano-Silber (Kontrolle);
**Figur** 6 einen vergrößerten Ausschnitt (rechteckiger Kasten) mit abgetöteten Hefezellen (dunkelgrau) sowie von einer Zelle vor dem Beginn der Färbung (helle Zelle); und
**Figur** 7 mikroskopische Abbildungen von Algenzellen rund um ein mit der antimikrobiellen Zusammensetzung beschichtetes Kügelchen ("Bead", dunkler Kreis),
   a) zu Beginn des Versuchs,
   b) nach 3 Minuten.

Beschreibung beispielhafter und bevorzugter Ausführungsformen der Erfindung

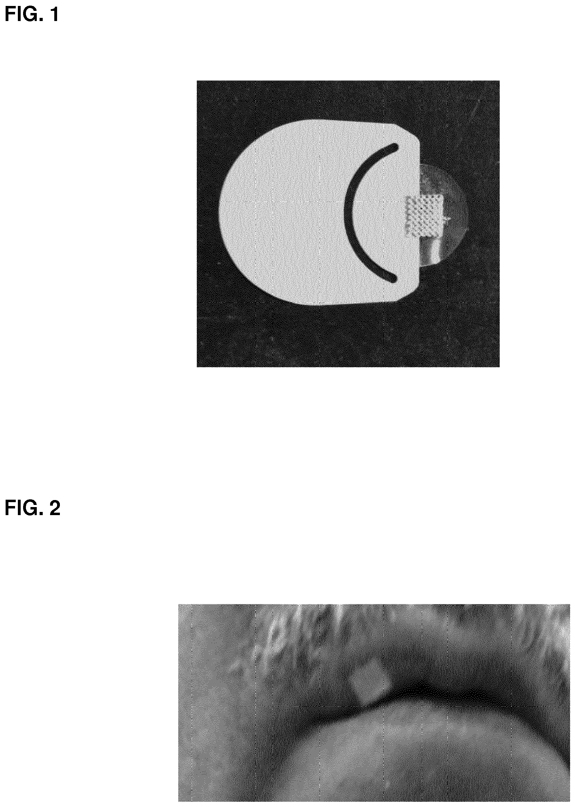

**Figur 1** zeigt eine fotographische Abbildung einer mit der antimikrobiellen Zusammensetzung beschichteten selbstklebenden Folie mit Hydrogel-Untergrund. Als Trägermaterial für die aktivierte antimikrobielle Ag/Ru-Zusammensetzung dient in diesem Ausführungsbeispiel eine selbstklebende Folie, mittels der die Zusammensetzung leicht auf eine zu behandelnde Oberfläche, z. B. die Haut, aufgebracht werden kann. Auf diese oder ähnliche Weise kann die antibakterielle Zusammensetzung in Form eines Wundschnellverbandes bzw. Pflasters appliziert werden. Der Hydrogel-Untergrund dient dabei der Speicherung von Feuchtigkeit, welche die Wirkung der antimikrobiellen Zusammensetzung fördert bzw. steigert.

**Figur 2** zeigt eine fotographische Abbildung eines applizierten Trägermaterials mit antimikrobieller Zusammensetzung. Das Trägermaterial wurde hier zum Zwecke der topischen Behandlung von Lippenherpes auf die Lippenhaut eines Menschen aufgebracht.

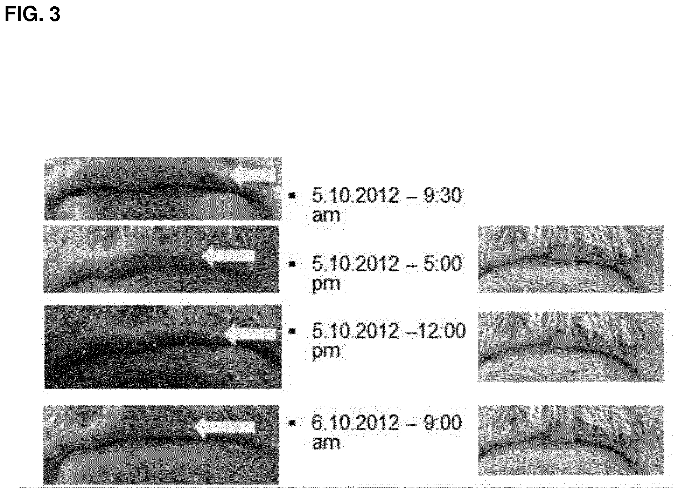

**Figur 3** zeigt fotographische Abbildungen des Heilungsverlaufs eines Lippenherpes unter der Behandlung mit der antimikrobiellen Zusammensetzung. Nach dem Auftreten der ersten Symptome eines beginnenden Lippenherpes wurde ein Trägermaterial mit aktivierter antimikrobieller Ag/Ru-Zusammensetzung auf die Lippenhaut eines Manschen aufgebracht (Abbildungen rechts) und der Verlauf der Erkrankung im Abstand weniger Stunden dokumentiert (Abbildungen links). Es stellte sich heraus, dass die erfindungsgemäße Verwendung der antimikrobiellen Zusammensetzung in topischer Anwendung auf der Haut bereits innerhalb von knapp 24 Stunden zu einem vollständigen Abklingen der Symptome führt. Durch Verwendung eines erfindungsgemäßen Herpespflasters gelingt eine deutliche Verkürzung des Krankheitsverlaufs, wobei die Stadien 2-5 des Herpesausbruchs bei rechtzeitiger Pflasterapplikation gar nicht mehr durchlaufen werden. Durch die Wiederverwendbarkeit des Trägermaterials während der Herpesbehandlung ergibt sich gegenüber den bisherigen Herpesprodukten ein klarer Vorteil. Beide Faktoren bedeuten für die Betroffenen einen deutlichen Zugewinn an Lebensqualität und Kostenersparnis.

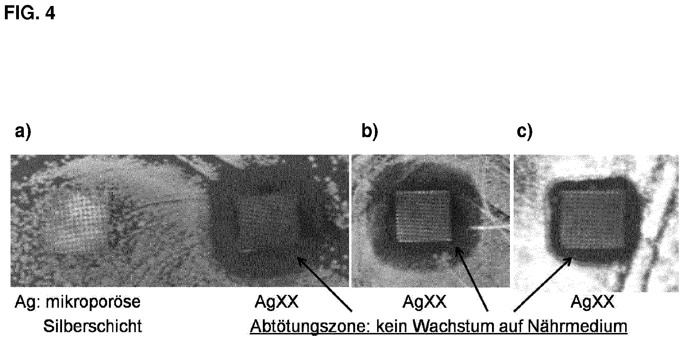

**Figur 4** zeigt fotographische Abbildungen von Nährböden mit Mikroorganismus-Kolonien rund um mit der antimikrobiellen Zusammensetzung beschichteten Trägermaterialien. Es zeigte sich hierbei, dass die antimikrobielle Zusammensetzung eine Breitbandwirkung gegen Bakterien (a: E. coli) und Pilze (b: pathogene Hefe und c: Pinselschimmel) hat. Eingesetzt wird die antimikrobielle Zusammensetzung hier in Form von beschichteten Drahtnetzen, die auf mit den jeweiligen Mikroorganismen besiedelte Nährböden gelegt wurden.

Nach einer Inkubation von 36 Stunden (106/ml, 30°C), zeigten sich rund um die erfindungsgemäßen Drahtnetzabschnitte deutliche Hemmhöfe, welche die antimikrobielle Wirkung der beschichten Drahtnetze belegen. Dagegen zeigte sich, dass ein mit einer mikroporösen Silberbeschichtung versehenes Drahtnetz (a (links): Kontrolle) die Keime nicht abtöten kann und vollständig überwachsen wird.

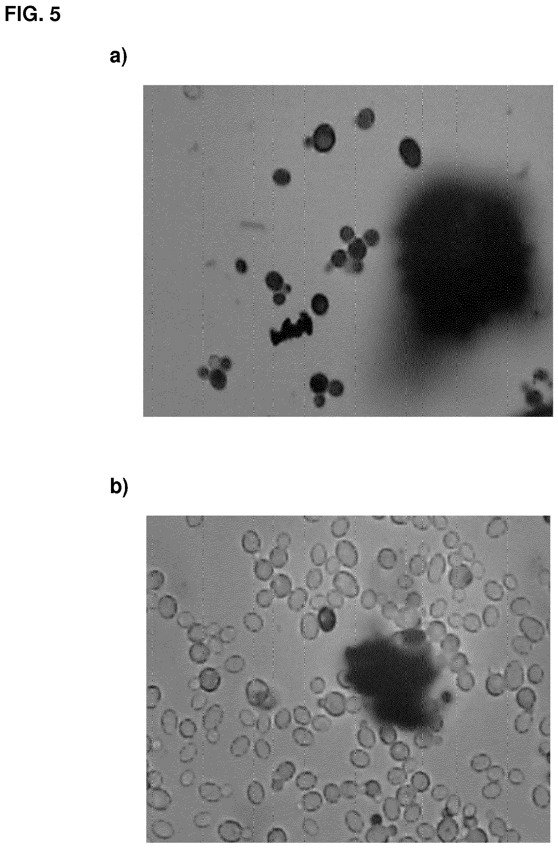

**Figur 5** zeigt abgetötete Hefepilze nach 15 Minuten Einwirkdauer einer pulverförmigen antibakteriellen Zusammensetzung (dunkler Bereich). Die wässrige Lösung, in der sich die Hefepilze befanden, enthielt einen Farbstoff (Methylenblau), der nur durch die Zellwand in die abgetöteten Zellen gelangt sein kann. Durch den in der Zelle veränderten pH-Wert reagiert der Farbstoff und bildet seine charakteristische blaue Farbe aus. In Figur 5a sind Hefepilze in den Kontakt mit aktiviertem Ag/Ru-Pulver gebracht worden. Nach 15 Minuten sind alle Hefepilze im Umfeld von ca. 50 µm um die pulverförmige antimikrobielle Zusammensetzung durch den Farbstoff Methylenblau gefärbt. Die Einfärbung zeigt, dass die Hefepilze abgetötet wurden, denn der Farbstoff kann nur in die Zelle gelangen, wenn die Zellen tot und ihre Zellwand geschädigt ist. Da der Färbevorgang einige Zeit in Anspruch nimmt, sind die Hefezellen in weniger als 15Minuten abgetötet worden. Im Gegensatz dazu sind durch Nano-Silber (Figur 5b: Kontrolle) selbst nach 60 Minuten keine Keime abgetötet worden. Die einzelnen toten Hefepilze sind bereits von Versuchsbeginn an abgetötet gewesen und zeigen lediglich an, dass in dieser wässrigen Lösung der Farbstoff für die abgetöteten Keime auch enthalten war.

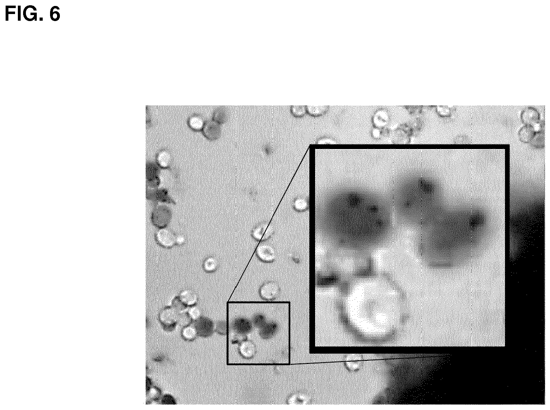

**Figur 6** zeigt einen vergrößerten Ausschnitt (rechteckiger Kasten) mit abgetöteten Hefezellen (dunkelgrau) sowie einer Zelle vor dem Beginn der Färbung (helle Zelle). Die dunklen Punkte in den Zellwänden können als Poren interpretiert werden. Da die Blaufärbung nach der Abtötung der Zellen einige Zeit in Anspruch nimmt, ist die in dem vergrößerten Ausschnitt zu sehende helle Zelle, wie anhand der ersten dunklen Punkte in der Membran abgelesen werden kann, möglicherweise bereits tot, aber noch nicht blau eingefärbt.

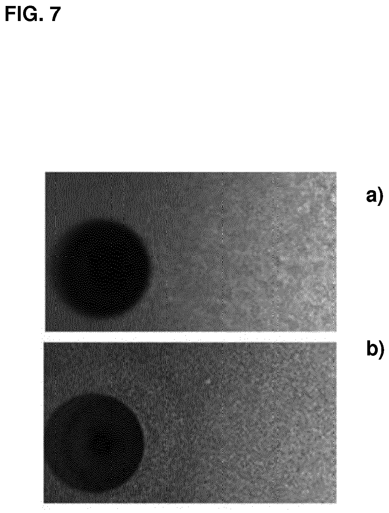

**Figur 7** mikroskopische Abbildungen von Algenzellen rund um ein mit der antimikrobiellen Zusammensetzung beschichtetes Kügelchen ("Bead", dunkler Kreis). Zu Beginn des Experiments (Figur 7a) sind die Algen intakt, während sich bereits 3 Minuten später tote Algenzellen rund um das Kügelchen zeigen (Figur 7b). Es zeigt sich also, dass die erfindungsgemäße Verwendung einer aktivierten Ag/Ru-Zusammensetzung auch zu einer Abtötung von Algenzellen führt.

In weiteren, hier nicht bildlich dargestellten Versuchen konnte auch gezeigt werden, dass die erfindungsgemäße Verwendung einer aktivierten Ag/Ru-Zusammensetzung auch eine antimikrobielle Wirkung gegen Legionellen hat.

Alle durchgeführten Versuche machen deutlich, dass die antimikrobielle Zusammensetzung offensichtlich zu einer Zerstörung der Zellwand und/oder Zellmembran der jeweiligen Mikroorganismen führt, welche zu einer irreversiblen Schädigung der Zellen führt und diese dadurch effektiv abtötet.

Trotz dieser starken antimikrobiellen Wirkung ist die erfindungsgemäße Verwendung der aktivierten Ag/Ru-Zusammensetzung für die damit behandelten Personen oder Tiere unbedenklich. In einer Studie zur Zelltoxizität mit antimikrobiell beschichteten Glaskugeln wurden Untersuchungen an MRC-5-Zelllinien durchgeführt. Die Untersuchungen wurden nach ISO 10993, ISO 10993-12 und USP Chapter 87 und 1031 durchgeführt. Die Untersuchung ergab einen zytotoxischen Effekt der Stufe 1 (leichte Zytotoxizität), der als unbedenklich gilt und eine Anwendung am Menschen zulässt.

Erste *in vivo* - Versuch an Ratten haben die Verträglichkeit der antimikrobiellen Zusammensetzung bestätigt. Die Versuche wurden mit aktivierten Ag/Ru-Glaskugeln in Blasen von Ratten durchgeführt. Es ergab sich kein zytotoxisch negativer Befund, der einer Verwendung der antimikrobiellen Zusammensetzung für urologische Zwecke entgegenstehen würde.

### Beispiel:

Verwendung der antimikrobiellen Zusammensetzung als Herpes - Pflaster:
Herpes-simplex-1 ist eine selbstlimitierende Erkrankung. Die antivirale Therapie ist nicht zwingend erforderlich. Viele Patienten, die unter Rezidiven leiden, konsultieren keinen Arzt und nehmen OTC-Präparate. Bei einer nahezu 90%-igen Durchseuchung der europäischen Bevölkerung mit Herpes simplex-Viren und einer jährlichen Inzidenz von bis zu 30 % der betroffenen Bevölkerung stellt sich immer wieder die Frage nach der effektivsten Therapie. Speziell Herpes simplex-Virus Typ I (HSV-1) ist weit verbreitet und belastet Betroffene durch die typischen Infektionen von Epithelzellen der Haut und assoziierter Neuronen in der Mundregion. Eine amerikanische Studie zeigte eine endogene Re-Infektion bei ca. 30% der infizierten Patienten, die rekurrierende Infektionsrate liegt bei ca. 1%.

Die Infektion spielt sich im Wesentlichen im Lippenbereich und Umgebung ab.

Die Herpes simplex-Erkrankung ist aufgrund der weiten Verbreitung nicht nur eine gesundheitliche, sondern auch ein soziale Belastung für die betroffenen Personen. Die bisher auf dem Markt befindlichen Herpes-Produkte führen lediglich zu einer geringen zeitlichen Verringerung des Krankheitsverlaufs und können allenfalls die Symptome des Lippenherpesausbruchs lindern.

Die vorteilhafte Möglichkeit, aktivierte Ag/Ru-Zusammensetzung auf die unterschiedlichsten Trägerwerkstoffe auftragen zu können, ermöglicht die Konzeption eines völlig neu aufgebauten Herpespflasters. Grundträger ist eine in der Medizintechnik handelsübliche mikroporöse Polymerfolie, die einseitig mit einem hautverträglichen Kleber versehen ist. Darauf wird ein dünner Film eines Hydrogels aufgebracht, das eine ausreichende Haftung eines mit aktiviertem Silber/Ruthenium beschichteten Trägermaterials (z. B. Gewebe) gewährleistet und als Feuchtigkeitsspeicher dient. Feuchtigkeit erhöht die Wirksamkeit der antimikrobiellen Zusammensetzung. Gleichzeitig lässt sich das Ag/Ru-Gewebe von dem Gel leicht wieder lösen, um es auf eine neue Trägerfolie zu applizieren, sobald die Haftung des Herpespflasters von der zu behandelnden Stelle verloren gegangen ist.

Das neuartige Ag/Ru-Pflaster wird in gleicher Weise wie bei den klassischen Pflastern auf die betroffene Stelle im Gesicht appliziert. Da die Wirksamkeit der antimikrobiellen Zusammensetzung durch Feuchtigkeit unterstützt wird, kann Feuchtigkeit auch durch Befeuchten mit Wasser von außen auf das feuchtigkeitsdurchlässige Pflaster und somit auf das aktivierte Ag/Ru-Gewebe und die Haut gebracht werden.

Ein Nachteil des Penciclovir-Wirkstoffes ist, dass er alle 2 Stunden appliziert werden muss. Die bekannten Herpespflaster haben auf der Herpesstelle eine Haftungszeit von maximal 8 h. Danach muss das Herpespflaster gewechselt werden. Beim erfindungsgemäßen Ag/Ru-Pflaster liegt die Haftungsdauer des Pflasters auf dem Herpes etwa in der gleichen Zeit. Allerdings wird das Wirkmedium (Ag/Ru-Gewebe) lediglich von einem Haftpflasterträger zum nächsten transferiert, ohne dass ein neues Ag/Ru-Gewebe benutzt werden muss. Das bedeutet, dass man während der gesamten Heilungsperiode mit nur einem Ag/Ru-Wirkmedium auskommen kann. Lediglich der Haftgrundträger wird erneuert, was einen deutlichen Kostenvorteil darstellt. Ferner ist die Anwendung sehr einfach durchzuführen.

In diversen Eigenversuchen konnte die hohe Wirksamkeit des erfindungsgemäßen Ag/Ru-Herpespflasters mehrfach bestätigt werden (siehe Figur 3).

Vorteile des erfindungsgemäßen Ag/Ru-Pflasters und dessen erfindungsgemäßer Verwendung:
- Kurze Anwendungsdauer bis zur erfolgreichen Herpeszurückdrängung,
- Einfache Handhabung,
- Keine Nebeneffekte zu erwarten,
- Im Vergleich zu den herkömmlichen Anti-Herpessystemen völlig neuer Wirkmechanismus,
- Das Ag/Ru-Herpespflaster basiert auf einem speziell aufgebauten Beschichtungssystems aus Edelmetallen und einem Vitaminderivat. Diese strukturierte Materialkombination verleiht dem aktivierten Ag/Ru-System eine sehr hohe Wirksamkeit gegen Mikroorganismen. Eine wichtige Rolle spielt dabei die Ausbildung eines mikroelektrischen Feldes an der Ag/Ru-Oberfläche, das die katalytische Wirkung der Ag/Ru-Oberfläche verstärkt.
- Die Ag/Ru-Oberfläche wird für die Herpespflaster in einem Rolle-zu-Rolle-Beschichtungsprozess weitgehend automatisiert hergestellt.

Alternativ zur Verwendung der antimikrobiellen Zusammensetzung in Form eines Wundschnellverbandes sind auch folgende Anwendungsformen möglich:
Salbe (einschl. Fettsalben), Creme, Lotion / Milch, Flüssigkeit (einschl. Lösung und/oder Suspension; auf wässriger, auf alkoholischer oder auf aromatischer Grundlage), Schüttelmixtur (Lotio), Feststoff (z.B. Pulver), Paste, Zäpfchen, Augentropfen oder Augensalben, oder als Mittel zur Lokaltherapie an Ohren, Nase und in der Mundhöhle in Tropfen- oder Sprayform.

## Patentansprüche

1. Antimikrobielle Zusammensetzung, welche metallisches Silber, metallisches Ruthenium sowie Ascorbinsäure oder ein Salz der Ascorbinsäure umfasst, zur Verwendung bei der topischen Behandlung oder Vermeidung von Erkrankungen der Haut, Hautanhangsgebilde oder Schleimhaut, die durch die Infektion mit mindestens einem Herpes simplex-Virus verursacht werden.

2. Antimikrobielle Zusammensetzung nach Anspruch 1, **dadurch gekennzeichnet, dass** das Virus das Herpes simplex-Virus Typ 2 ist.

3. Antimikrobielle Zusammensetzung nach Anspruch 1 oder 2, **dadurch gekennzeichnet, dass** das Virus das Herpes-Simplex-Virus Typ 1 ist.

4. Antimikrobielle Zusammensetzung nach einem der vorhergehenden Ansprüche, **dadurch gekennzeichnet, dass** die antimikrobielle Zusammensetzung auf mindestens eine Oberfläche eines Trägermaterials aufgebracht ist.

5. Antimikrobielle Zusammensetzung nach Anspruch 4, **dadurch gekennzeichnet, dass** das Trägermaterial ein Gewebe, eine Folie, ein Netz, ein Verbandmaterial, eine Kompresse oder ein Wundschnellverband ist.

6. Antimikrobielle Zusammensetzung nach Anspruch 4 oder 5, **dadurch gekennzeichnet, dass** das Trägermaterial benetzbar und/oder durchlässig für Feuchtigkeit ist.

7. Antimikrobielle Zusammensetzung nach einem der Ansprüche 4 bis 6, **dadurch gekennzeichnet, dass** das Trägermaterial ein Material zur Speicherung von Feuchtigkeit umfasst.

8. Antimikrobielle Zusammensetzung nach einem der Ansprüche 4 bis 7, **dadurch gekennzeichnet, dass** das Trägermaterial gas- und/oder luftdurchlässig ist.

9. Antimikrobielle Zusammensetzung nach einem der vorhergehenden Ansprüche, **dadurch gekennzeichnet, dass** die antimikrobielle Zusammensetzung mindestens eine Silberschicht mit mindestens einer darauf aufgebrachten Ruthenium-Schicht umfasst, wobei die Silberschicht von der Ruthenium-Schicht in mindestens einem Teilbereich unbedeckt ist.

10. Antimikrobielle Zusammensetzung nach einem der vorhergehenden Ansprüche, **dadurch gekennzeichnet, dass** die antimikrobielle Zusammensetzung mindestens einen Silberdraht oder zumindest silberbeschichteten Draht umfasst, der teilweise mit einer Ruthenium-Schicht bedeckt ist.

11. Antimikrobielle Zusammensetzung nach einem der vorhergehenden Ansprüche, **dadurch gekennzeichnet, dass** die antimikrobielle Zusammensetzung Silber-Ruthenium-Bimetall-Partikel umfasst.

12. Antimikrobielle Zusammensetzung nach einem der vorhergehenden Ansprüche, **dadurch gekennzeichnet, dass** die antimikrobielle Zusammensetzung in einer Suspension, einer Creme, einer Salbe, einem Gel oder einem Pulver enthalten ist.

13. Verbandmaterial oder Wundschnellverband zur Verwendung bei der topischen Behandlung oder Vermeidung von Erkrankungen der Haut, Hautanhangsgebilde oder Schleimhaut, die durch die Infektion mit mindestens einem Herpes simplex-Virus verursacht werden, **dadurch gekennzeichnet, dass** dieses oder dieser eine antimikrobielle Zusammensetzung umfasst, welche metallisches Silber, metallisches Ruthenium sowie Ascorbinsäure oder ein Salz der Ascorbinsäure umfasst.

## Claims

1. Antimicrobial composition comprising metallic silver, metallic ruthenium, and ascorbic acid or a salt of ascorbic acid, for use in the topical treatment or prevention of skin, skin adnexa or mucosa diseases which are caused by infection with at least one Herpes simplex virus.

2. Antimicrobial composition according to claim 1, **characterised in that** the virus is Herpes simplex virus Type 2.

3. Antimicrobial composition according to claim 1 or 2, **characterised in that** the virus is Herpes simplex virus Type 1.

4. Antimicrobial composition according to any one of the preceding claims, **characterised in that** the antimicrobial composition is applied onto at least one surface of a carrier material.

5. Antimicrobial composition according to claim 4, **characterised in that** the carrier material is a tissue, a foil, a mesh, a dressing material, a compress, or a first-aid wound dressing.

6. Antimicrobial composition according to claim 4 or 5, **characterised in that** the carrier material is wettable and/or permeable for moisture.

7. Antimicrobial composition according to any one of claims 4 to 6, **characterised in that** the carrier material comprises a material for retaining moisture.

8. Antimicrobial composition according to any one of claims 4 to 7, **characterised in that** the carrier material is permeable for gas and/or air.

9. Antimicrobial composition according to any one of the preceding claims, **characterised in that** the antimicrobial composition comprises at least one silver layer with at least one ruthenium layer applied thereon, wherein the silver layer is not covered by the ruthenium layer in at least one subzone.

10. Antimicrobial composition according to any one of the preceding claims, **characterised in that** the antimicrobial composition comprises at least one silver wire or at least silver-coated wire, which is partly covered by a ruthenium layer.

11. Antimicrobial composition according to any one of the preceding claims, **characterised in that** the antimicrobial composition comprises silverruthenium bimetallic particles.

12. Antimicrobial composition according to any one of the preceding claims, **characterised in that** the antimicrobial composition is included in a suspension, a cream, an ointment, a gel, or a powder.

13. Dressing material or first-aid wound dressing for use in the topical treatment or prevention of skin, skin adnexa or mucosa diseases which are caused by infection with at least one Herpes simplex virus, **characterised in that** this comprises an antimicrobial composition comprising metallic silver, metallic ruthenium, and ascorbic acid or a salt of ascorbic acid.

## Revendications

1. Composition antimicrobienne, laquelle comprend de l'argent métallique, du ruthénium métallique, ainsi que de l'acide ascorbique ou un sel de l'acide ascorbique, destinée à l'utilisation pour le traitement topique ou la prévention de troubles de la peau, des phanères ou des muqueuses, qui sont provoqués par l'infection par au moins un virus de l'herpès simplex.

2. Composition antimicrobienne selon la revendication 1, **caractérisée en ce que** le virus est celui de l'herpès simplex de type 2.

3. Composition antimicrobienne selon la revendication 1 ou 2, **caractérisée en ce que** le virus est celui de l'herpès simplex de type 1.

4. Composition antimicrobienne selon l'une quelconque des revendications précédentes, **caractérisée en ce que** la composition antimicrobienne est appliquée sur au moins une surface d'une matière porteuse.

5. Composition antimicrobienne selon la revendication 4, **caractérisée en ce que** la matière porteuse est un tissu, un film, un filet, une matière à pansement, une compresse ou un pansement rapide.

6. Composition antimicrobienne selon la revendication 4 ou 5, **caractérisée en ce que** la matière porteuse est mouillable et/ou perméable à l'humidité.

7. Composition antimicrobienne selon l'une quelconque des revendications 4 à 6, **caractérisée en ce que** la matière porteuse comprend une matière destinée à accumuler de l'humidité.

8. Composition antimicrobienne selon l'une quelconque des revendications 4 à 7, **caractérisée en ce que** la matière porteuse est perméable au gaz et/ou à l'air.

9. Composition antimicrobienne selon l'une quelconque des revendications précédentes, **caractérisée en ce que** la composition antimicrobienne comprend au moins une couche d'argent avec au moins une couche de ruthénium appliquée sur celle-ci, dans au moins une zone partielle, la couche d'argent n'étant pas recouverte par la couche de ruthénium.

10. Composition antimicrobienne selon l'une quelconque des revendications précédentes, **caractérisée en ce que** la composition antimicrobienne comprend au moins un fil d'argent ou au moins un fil revêtu d'argent, qui est partiellement recouvert d'une couche de ruthénium.

11. Composition antimicrobienne selon l'une quelconque des revendications précédentes, **caractérisée en ce que** la composition antimicrobienne comprend des particules bimétalliques d'argent-ruthénium.

12. Composition antimicrobienne selon l'une quelconque des revendications précédentes, **caractérisée en ce que** la composition antimicrobienne est contenue dans une suspension, une crème, une pommade, un gel ou une poudre.

13. Matière à pansement ou pansement rapide, destiné à l'utilisation pour le traitement topique ou la prévention de troubles de la peau, des phanères ou des muqueuses, qui sont provoqués par l'infection par au moins un virus de l'herpès simplex, **caractérisée en ce que** celle-ci ou celui-ci comprend une composition antimicrobienne laquelle comprend de l'argent métallique, du ruthénium métallique, ainsi que de l'acide ascorbique ou un sel de l'acide ascorbique.
